Europäisches Patentamt

European Patent Office

Office européen des brevets

(19)

(11) Publication number: **0 228 225**
**A2**

(12) **EUROPEAN PATENT APPLICATION**

(21) Application number: 86309757.2

(22) Date of filing: 15.12.86

(51) Int. Cl.⁴: **G01N 33/543** , //G01N33/547

(30) Priority: 16.12.85 US 808987

(43) Date of publication of application:
08.07.87 Bulletin 87/28

(84) Designated Contracting States:
AT BE CH DE FR GB IT LI LU NL SE

(71) Applicant: ORTHO DIAGNOSTIC SYSTEMS INC.
Route 202
Raritan New Jersey 08669(US)

(72) Inventor: Finkenaur, Amy L.
10 Steven Avenue
Somerville, N.J. 08876(US)

(74) Representative: Jones, Alan John et al
CARPMAELS & RANSFORD 43 Bloomsbury Square
London, WC1A 2RA(GB)

(54) Immunoassay kit and method employing modified solid surface.

(57) An immunoassay kit and method is described. The kit includes a solid surface having immobilized thereon a biologically specific substance for detecting the presence of an analyte. The substance is covalently bonded to the solid surface by a difunctional hydrophilic group having a molecular chain length of about 10 to about 300 Angstroms.

EP 0 228 225 A2

# IMMUNOASSAY KIT AND METHOD EMPLOYING MODIFIED SOLID SURFACE

The invention relates to an immunoassay kit and method employing an improved solid surface.

## Background of the Invention

A number of immunoassays are carried out by selectively immobilizing a biologically specific substance on a solid surface, and then detecting the presence of that substance on the surface by any of several procedures. For instance, one version of the Enzyme-Linked Immunosorbent Assay - ("ELISA"), is carried out as follows:

A biologically specific substance that will selectively bind to the unknown to be analyzed is immobilized on a solid surface, usually polystyrene or polyvinyl chloride. The remainder of the solid surface may then be blocked with a nonreactive molecule (a blocking buffer) to deter adsorption of biological molecules on the surface. The surface is then contracted (incubated) with a material that is suspected of containing the unknown. If any of the unknown is present, it will selectively bind to the biologically specific substance. The surface is then contacted with a predetermined cross-homologous antispecies ("detecting") antibody conjugated with an enzyme. (The cross-homologous antispecies antibody will selectively bind only to the unknown.) This may be done concurrently with the incubation with the unknown or as a separate step. After washing, the presence of the enzyme is detected by known procedures. For instance, if the enzyme is a peroxidase, it is used to oxidize a secondary substrate (such as o-phenylenediamine) to a colored product. The intensity of the color thus produced is proportional to the concentration of unknown in the original sample tested.

In a Radioimmune Assay ("RIA"). the principle is the same, except that the detecting antibody is radioactively labeled, and the radioactivity of the surface after washing is a measure of the concentration of the unknown in the sample assayed.

In an Agglutination Assay, the biologically specific substance is physically immobilized on the surface of a latex particle in a latex dispersion. If . the unknown is present, the latex will agglutinate and the dispersion will become cloudy. This test is most useful as a qualitative ("yes" or "no") test for the presence of the unknown, and is rarely used for quantitative analyses.

In many cases, the unknown being analyzed for is present, if at all, in a very low concentration. In such a case, it is desirable for the assay procedure to be sensitive enough to reliably detect the unknown at low concentrations. To this end, the immobilized biologically specific substance should be as reactive as possible. This invention is directed to a means for enhancing the reactivity of a biologically specific substance immobilized on a surface, to thereby increase the sensitivity of an immunoassay utilizing said immobilized and in particular, to a means for enhancing the stability of the biologically active surface so that its activity remains substantially unchanged over a significant period of time.

## Brief Summary of the Invention

An immunoassay kit including a solid surface having immobilized thereon a biologically specific substance for detecting the presence of an analyte, wherein said substance is covalently bonded to said surface by a difunctional hydrophilic group having a molecular chain length of from about 10 to about 300 Angstroms.

The invention also provides an immunoassay method which employs said kit.

## The Prior Art

Hellerman et al., Makromol. Chem. 184, 2603-2617(1983), disclose poly(ethylene glycol)s grafted onto cross-linked polystyrene as a new class of multidetachably immobilized hydrophilic macromolecular supports for the synthesis of peptides.

Rotmans et al., Journal of Immunological Methods, 57, 87-98(1983), disclose the use of - (hydrophobic) suberic acid spacer molecules to couple a hapten onto the surface of aminated polystyrene in an ELISA.

Rembaum et al., in U.S. Patent No. 4,224,198, disclose the use of (hydrophobic) diaminoheptane and e-aminocaproic acid to couple biologically specific substances to the surface of microspheres.

Fischer, in U.S. Patent No. 4,264,766, disclose the use of a polyfunctional carbohydrate or other polyhydroxy compound to link a bioactive material to a latex particle.

Von Stetten et al., in U.S. Patent No. 4,273,865, disclose a layer of silicic acid on a base which is used to couple or covalently bond a biochemical material.

Brenna et al., in European Patent Application No. 0,131,546, disclose a method for stably binding antigens and allergens to a polystyrene surface, utilizing a polyfunctional aldehyde.

Sakakibara et al., in U.S. Patent No. 4,341,758, disclose the use of polyacrylic acid as a linking group on the surface of a latex particle in an immunoassay method. Hosaka et al., in U.S. Patent No. 4,418,152, discloses the use of a 1,2-dihydroxyprop-3-oxy group as a coupler to the surface of a latex particle in an agglutination assay.

Ozkan, in U.S. Patent No. 4,450,231, discloses the use of a layer of polyethylene glycol of molecular weight 2000 to 20,000 as a base for absorbing immune complexes in an immunoassay.

Detailed Description of the Invention

The principal novelty of this invention resides in the use of a difunctional hydrophilic spacer to covalently bond a biologically specific substance to a solid surface in an immunoassay. The solid surface can be any polymeric material onto which the spacer can be covalently bonded. Thus, the polymeric material should contain reactive groups which can bond to the spacer molecule. Such reactive groups include amino, especially primary amino, carboxyl, hydroxyl, amido, halo, haloalkyl - (preferably chloromethyl), isocyanato, and the like, to which the spacer molecule can be covalently bonded by known chemical reactions. Specific illustrative polymeric materials that can be used for the surface include styrene/chloromethylstyrene copolymer, styren/maleic anhydride copolymer, polyvinyl chloride, aminated polystyrene, styrene/butadiene/chloromethylstyrene terpolymer, acrylic polymers containing polymerized acrylic or methacrylic acid, acrylamide, or hydroxyethyl methacrylate, chloroprene or the like.

In many cases, the polymeric material will be employed as a coating on a base of another material such as a polystyrene or a polyvinyl chloride microtitration tray, sampling stick, or the like. In other cases, the polymeric material will be employed as a latex for use in an agglutination assay. The specific form of the polymeric solid surface will be determined by the particular type of assay intended, the selection of which is well known in the art.

The difunctional hydrophilic spacer molecule that constitutes the principal novelty of the invention can be derived from a hydrophilic material such as a polyoxyethylene glycol, a polyoxyethylene/oxypropylene glycol wherein the ratio of oxyethylene to oxypropylene groups is such that the material is hydrophilic, (i.e., is either water-soluble or is capable of absorbing water on the surface of the molecule), polyvinyl pyrrolidone, and other water-soluble or water-absorbable, low molecular weight polymers. These polymers are "difunctional". That is, they contain only two reactive groups -one to covalently bond to the solid surface, and the other to covalently bond to the biologically specific substance. Preferably, the two reactive groups on the hydrophilic spacer material are terminal groups. Preferably, the hydrophilic spacer material has terminal primary amino groups, although other reactive groups may be present. Such other reactive groups include thiol, hydroxyl, amino, carboxyl, and ester groups.

The spacer is coupled to the polymer surface by known types of chemical reaction. For instance, when the terminal group on the spacer is a primary amino group and the pendant reactive group on the polymer surface is a chloromethyl group, the solid polymer containing the chloromethyl group is simply contacted with an aqueous solution of the amine-terminated spacer material, preferably at moderately elevated temperatures such as 50°C., for one to twenty-four hours, and the reaction between the amino and chloromethyl groups occurs as follows:

$NH_2$-SPACER-$NH_2$ + -Ph-$CH_2$Cl—-Ph-$CH_2$-NH-SPACER-$NH_2$ + HCl

wherein Ph represents phenylene, as from a styrene/chloromethylstyrene copolymer.

Another type of reaction than can be used to covalently bond the spacer to the surface is the reaction of primary amino with carboxylic anhydride to form amido groups. This can be used, e.g., to bond primary amine-terminated spacer to a styrene/maleic anhydride copolymer by simply contacting an aqueous solution of the spacer with the copolymer surface at 37° C. for 24 hours.

The hydrophilic spacer materials will normally have molecular weights such that they have a chain length of from about 10 to about 300 Angstroms, which corresponds approximately to about 6 to 150 atoms in the chain.

Specific illustrative spacer materials include primary amine-terminated polyoxyethylene/oxypropylene compositions of the formula:

$NH_2$-[CH(CH_3)CH_2O]_a-[CH_2CH_2O]_b-[CH_2CH(CH_3)O]_c-CH_2CH(CH_3)-NH_2$

wherein a, b and c are numbers having average values such that the ratio of (a + c) to b is such that the composition is hydrophilic, and wherein the total of a + b + c is such that the chain length of the molecule is from about 10 to about 300 Angstroms.

The selection of the biologically specific substance is governed by the identity of the substance to be analyzed, or analyte. Thus, the biologically specific substance must have biological specificity for the analyte. The types of materials that can serve as biologically specific substances include peptides, proteins, cell wall material, viral particles, enzymes, bacterial antigens, nucleic acids, DNA, RNA, carbohydrates, antibodies, and the like.

The biologically specific substance can be covalently bonded to the spacer molecule by known types of reaction. For example, a biologically specific substance containing a free carboxyl group can be coupled to an amine-terminated spacer by using a carbodiimide to catalyze the reaction between -COOH and -NH₂ to give an amide (peptide) bond. Another type of reaction by which a biologically specific substance can be bonded to the spacer molecule is to employ a protein such as an antibody originally having a disulfide bridge, cleaving the bridge by known techniques such as reduction by beta-mercaptoethanol, and then coupling the cleaved protein fragment to a spacer having a thiol end group, such as a polyethylene oxide having a thiol end group. The two thiol groups will react on standing to form a disulfide bridge, in the absence of reducing agents. The advantage of this technique is to insure preservation of the antibody active site, while giving superior orientation of the biologically specific substance (i.e., the active site is spacially oriented away from the solid surface for better reactivity towards the analyte). Another means for coupling the biologically specific substance to the spacer is to employ gluteraldehyde or other difunctional aldehyde to couple an amine-terminated spacer to a biologically specific substance containing a free primary amino group. A wide variety of other bifunctional cross-linking reagents are commercially available for such biochemical coupling. They include imidoesters, N-hydroxysuccinimide esters, azides, and the like.

After the biologically specific substance is bonded to the spacer, it is preferred to block the surface with a blocking buffer such as normal goat serum (i.e., serum from healthy goats), human albumin, bovine serum albumin, horse serum albumin, or other inert protein, which then prevents non-specific binding to the polymer surface.

The invention can be used for a wide variety of biological assays, so long as the assay utilizes the surface of a solid for detecting the presence of a specific biological substance. Thus, the invention can be used in ELISA, RIA and agglutination assays. As a general rule, the analyte will be contained in an aqueous medium.

The following is a partial listing of the types of assays that can be carried out in accordance with the invention, with a description of the type of biologically specific substance that is bonded to the spacer molecule and the corresponding analyte whose presence it is used to detect:

Peptides can be used as the biologically active substance to detect the presence of antibodies. For instance, a peptide from Epstein-Barr Virus Nuclear Antigen (EBNA) can be used to detect antibodies in serum, plasma, or blood, which arise during infectious mononucleosis(IgM). Peptides from HTLV III Human Transforming Virus can be used to detect antibodies to the native virus in AIDS patients.

Proteins can be used as the biologically active substance to detect antibodies. For instance, insulin can be used to detect antibodies to insulin in diabetics (specifically where bovine or other species of insulin is used).

Viral particles can be used as the biologically active substance to detect antibodies. For instance, Feline Leukemia Virus particles can be used to detect FeLV antibodies in cat blood, serum, or plasma.

Hormones can be used as the biologically active substance to detect antibodies. For instance, Thyroid Stimulating Hormone (TSH) can be used to detect antibodies to TSH in Grave's Disease.

Bacterial antigens can be used as the biologically active substance to detect antibodies to the bacteria. For instance, pili from gonococcus of N. gonorrhea can be used to detect antibodies in the infected human.

Nucleic acids, DNA, or RNA, can be used as the biologically active substance to detect genetic defects, autoimmune disease, bacteria, or viruses, as follows:

For genetic defects, a predetermined nucleic acid is used as the biologically active substance. DNA from the cells to be analyzed is denatured - (i.e., the two DNA strands are separated). If the DNA is the specific one for which the assay kit was made, it will couple (hybridize) to the said predetermined nucleic acid, and can then be detected using either a labeled probe or antibody. (A probe, in this case, would be a labelled nucleic acid having a predetermined sequence of bases such that it will couple to the exposed DNA analyte):

for autoimmune disease, a piece of DNA, RNA, or a synthetic polynucleotide is coupled to the surface to detect antibodies to the substance coupled, e.g., in the detection of lupus erythematosus; and

For bacteria or viruses, a nucleic acid coupled to the surface can be used to detect denatured bacterial or viral DNA from a sample. The DNA is then detected using a labelled nucleic acid probe.

A carbohydrate coupled to a surface can be used to detect an antibody to that carbohydrate. For instance, the mucopolysaccharide of carcinoembryonic antigen can be used to detect antibodies to this tumor antigen;

Antibodies can be used to detect other antibodies. For instance, polymerized human gamma globulin can be used to detect antibodies which arise in rheumatoid arthritis.

Also, the reverse of all of the above can be used. Thus, an antibody can be used as the biologically active substance to detect a peptide, protein, bacterial antigen, viral material, nucleic acid, carbohydrate, or a hormone. For instance, $\alpha$-FeLV can be used to detect the virus in blood or serum, $\alpha$-progesterone can be used to detect progesterone in urine, blood, saliva, milk, etc., and $\alpha$-$\beta$-human chorionic gonadotropin ($\beta$-HCG) can be used to test for this protein in urine to test for pregnancy. The following examples illustrate the invention:

Example 1

Synthesis of Poly(styrene-co-chloromethylstyrene) (S/CMS-70/30)

Freshly distilled styrene (85.62 grams, 0.82 mole) and chloromethylstyrene (31.34 grams, 0.205 mole) were combined with 0.158 gram pure 2,2'-azobis(2-methylpropionitrile), (recrystallized from a saturated methanol solution) in a 250 milliliter round bottom flask. The mixture was degassed by two cycles of freezing, evacuation, and thawing and then blanketed with nitrogen. The polymerization was performed at 60°C. for 18 hours. The polymer mass was frozen in liquid nitrogen and then deglassed. The polymer was dissolved in methylene chloride and precipitated into methanol. The purified polymer was isolated by filtration and dried in vaccuo at 60°C. for 72 hours. Elemental analysis of the copolymer indicated a composition of 70% styrene and 30% chloromethylstyrene, by mol per cent. (C: 85.67%, H: 7.36%)

Example 2

Polymer Coating and Jeffamine Treatment

Poly(styrene) sticks were dipcoated in a 5% S/CMS(70/30) polymer solution in methylene chloride and air dried. Sticks were placed in a beaker containing a 50% solution of Jeffamine ED600 in distilled water and heated to 50°C. for 24 hours in a water bath. Sticks were thoroughly washed with water and then air dried. Jeffamine ED 600 is a material that can be represented by the formula:
$NH_2$-$C_3H_6$-$(OC_3H_6)_a$-$(OC_2H_4)_b$-$(OC_3H_6)_c$-$NH_2$
wherein a + c = 2.5
b = 8.5
total length $\approx$50A

The presence of the Jeffamine ED600 on the surface of the stick is confirmed by low water contact angle present with the coated stick. The uncoated stick has a high water contact angle.

Example 3

ELISA Test For Feline Leukemia Virus

Materials Used:

Coated polystyrene sticks prepared as in Example 2, coated with copolymer of styrene and chloromethylstyrene plus the hydrophilic spacer Jeffamine ED 600.

Monoclonal antibody to feline leukemia virus - ("FeLV"), (i.e., a monoclonal antibody-"MoAB"-that is specific for FeLV), produced by well known hybridoma techniques, 9.3 mg/ml concentration in ammonium sulfate cut of ascites fluid (MoAB to FeLV).

Coupling buffer -Phosphate buffered saline - ("PBS"), pH 5.5, containing 1-ethyl-3-(3-dimethylaminopropyl)carbodiimide HCl ("EDC"), at a concentration of $6.2 \times 10^{-4}$Molar (0.119/liter).

Blocking buffer -10% normal goat serum in PBS at pH 7.4.

FeLV positive and negative reference sera, i.e., serum from an animal which is known to be positive or negative for feline leukemia virus.

MoAB to FeLV conjugated with horseradish peroxidase ("HRPO").

Ortho-phenylenediamine ("OPD") solution, 3 ml distilled water, one 40 mg OPD tablet with 12.5 $\mu l$ 3% hydrogen peroxide. (One 40 mg OPD tablet, containing 2 mg OPD in buffers, is dissolved in 3 ml distilled water and 12.5 $\mu l$ of a 3% $H_2O_2$ solution is added.)

2N aqueous HCL.

II. Method of coupling MoAB to FeLV to stick:

A. MoAb to FeLV is diluted in EDC-PBS coupling buffer, to a concentration of 50 $\mu g$/m l; Add 300 $\mu l$ of this diluted MoAB to FeLV to 12 x 75mm disposable glass tubes and incubate with Jeffamine-coated polystrene sticks overnight at 4°C. The EDC thereby catalyzes the reaction of primary amino groups from the Jeffamine with free carboxyl on the MoAB to form amide bonds.

B. Rinse sticks with distilled $H_2O$, then incubate in 500 $\mu l$ blocking buffer (10% normal goat serum in PBS) for ninety minutes at 37°C. The blocking buffer is used to prevent nonspecific interactions between the serum antibodies and the surface of the stick.

C. Dry sticks for one hour at 37°C.

III. Immunoassay procedure:

A. Add 50 $\mu l$ of positive or negative reference serum (or an unknown serum) to 10 x 75 mm glass tube.

B. Add 50 $\mu l$ of MoAB to FeLV-HRPO conjugate to the serum. (This is the MoAB to FeLV that has been labelled with horse radish peroxidase.)

C. Add sticks to the mixture of A and B and incubate 10 minutes at room temperature. If the serum contained the analyte (FeLV), it will bind to the MoAB. The labelled MoAB will also bind to the FeLV.

D. Wash sticks 3 times with 500 ml of distilled water in separate beakers. The washing step will remove all of the labelled MoAB except for that which binds to the analyte (FeLV) that is bound to the MoAB bonded to the stick.

E. Make up OPD solution just prior to wash step and add 100 $\mu l$ to each washed stick in a clean test tube.

F. Color change occurs in 5 to 20 minutes. Stop reaction with 50 $\mu l$ 2N HCl after 10 minutes and observe color. If labelled MoAB is bound to the stick, the horseradish peroxidase will effect a color change (from clear to blue) in the OPD solution that is readily detected spectrophotometrically.

**Claims**

1. An immunoassay kit including a solid surface having immobilized thereon a biologically specific substance for detecting the presence of a predetermined biological analyte, wherein said biologically specific substance is covalently bonded to said surface by a difunctional hydrophilic group having a molecular chain length of from about 10 to about 300 Angstroms.

2. The immunoassay kit of claim 1 wherein said difunctional hydrophilic group comprises a polyoxyethylene group.

3. The immunoassay kit of claim 1 or claim 2 wherein said biologically specific substance is a peptide, protein, cell wall material, viral particle, enzyme, bacterial antigen, a nucleic acid, an antibody, DNA, RNA, or a carbohydrate.

4. The immunoassay kit of any one of claims 1 to 3 wherein said biologically specific substance is bonded to said difunctional hydrophilic group by an amide group, a disulphide group or a secondary amino group.

5. The immunoassay kit of any one of claims 1 to 4 wherein said solid surface comprises a styrene/maleic anhydride copolymer.

6. The immunoassay kit of claim 5 wherein said difunctional hydrophilic group is bonded to said solid surface by a secondary amino group.

7. The immunoassay kit of any one of claims 1 to 4 wherein said solid surface comprises a styrene/maleic anhydride copolymer.

8. A method for detecting for the presence of a predetermined biological analyte which comprises the steps of:

a) incubating a material to be analyzed with a solid surface having immobilized thereon a first biologically specific substance that will selectively bind to said analyte, said first biologically specific substance being covalently bonded to said surface by a difunctional hydrophilic group having a molecular chain length of from about 10 to about 300 Angstroms;

b) incubating said surface with a labelled biologically specific substance that will bind only to said analyte;

c) washing said surface after step b); and

d) analyzing for the presence of the label on said labelled biologically specific substance.

9. The process of claim 8 wherein said process comprises an Enzyme-Linked Immunosorbent Assay, a Radioimmune Assay, or an agglutination assay.

10. The process of claim 8 or claim 9 wherein said hydrophilic group comprises a polyoxyethylene group.